# EUROPEAN PATENT APPLICATION

(11) **EP 3 103 795 A1**
(43) Date of publication of application: **14.12.2016**
(21) Application number: 16001185.4
(22) Date of filing: 24.05.2016
(51) Int. Cl.: C07D 209/60, A61K 49/22

(54) **COMPOUND OR SALT THEREOF AND PHOTOACOUSTIC IMAGING CONTRAST AGENT CONTAINING COMPOUND OR SALT THEREOF**

(30) Priority: 12.06.2015 JP 2015119747
(71) Applicant: Canon Kabushiki Kaisha, Tokyo (JP)
(72) Inventor: Fuku, Tatsuki, Tokyo (JP); Sasaguri, Daisuke, Tokyo (JP); Takahashi, Atsushi, Tokyo (JP); Mizusawa, Keigo, Tokyo (JP); Yuasa, Satoshi, Tokyo (JP)
(74) Representative: WESER & Kollegen

(57) **Abstract**

A compound or a salt thereof in which polyethylene glycol is bonded to a specific cyanine dye, according to any of formulae (1) (5), (6) or (7).

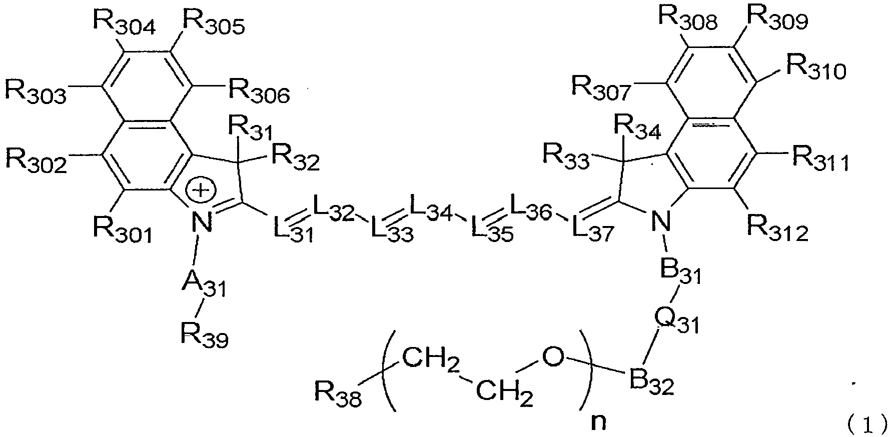

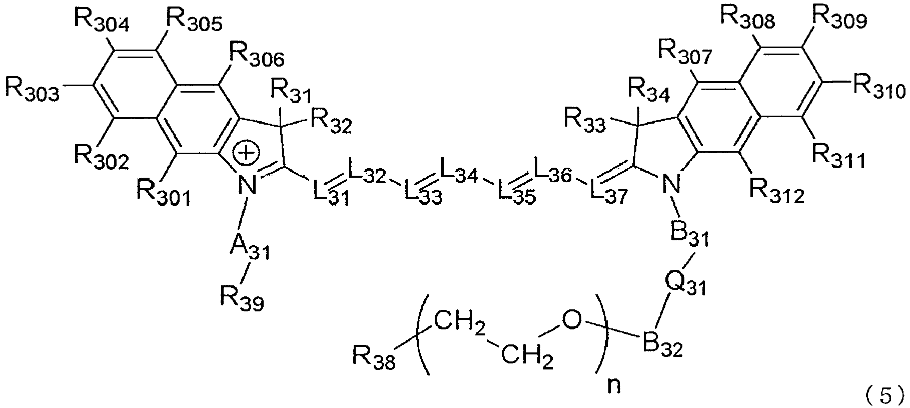

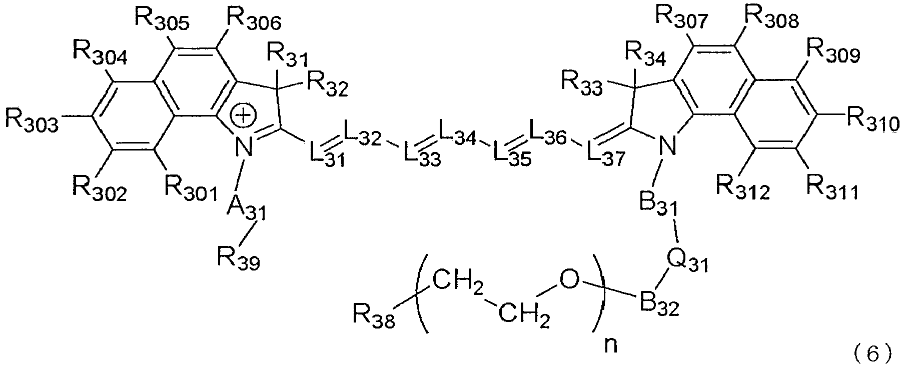

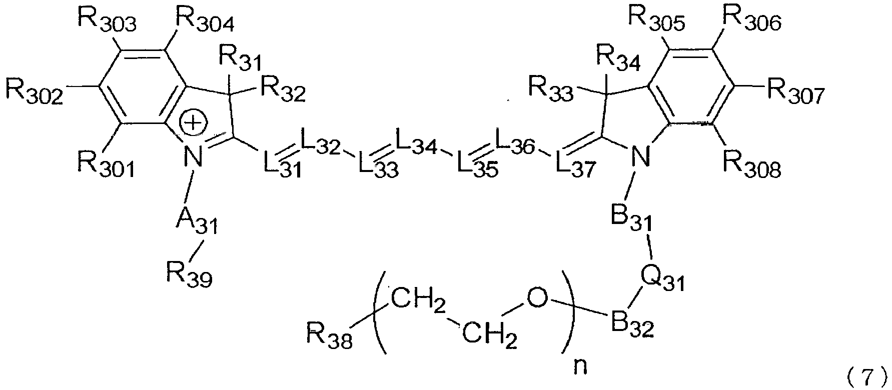

## Description

### BACKGROUND OF THE INVENTION

### Field of the Invention

The present invention relates to a compound or a salt thereof and a photoacoustic imaging contrast agent containing the compound or a salt thereof.

### Description of the Related Art

In recent years, a fluorescent imaging method and a photoacoustic imaging method have received attention as optical imaging methods suitable for use in performing noninvasive diagnoses.

The fluorescent imaging method is a widespread method in which a fluorescent dye is irradiated with light, and fluorescence emitted from the dye is detected. The photoacoustic imaging method in which the intensity of an acoustic wave generated by volume expansion caused by heat released from a molecule of an object of measurement irradiated with light and the position of generation of the acoustic wave are detected so that an image of the measurement object can be obtained. In the fluorescent imaging method and the photoacoustic imaging method, it is possible to use a dye as a contrast agent for increasing the intensity of the fluorescence and the intensity of the acoustic wave from the measurement object area.

PCT Japanese Translation Patent Publication No. 2012-520856 discloses that a junction body, in which a cyanine dye having a benzene ring as an aromatic structure is covalent bonded to polyethylene glycol, is used as an optical imaging agent.

However, the present inventors found that the junction body disclosed in PCT Japanese Translation Patent Publication No. 2012-520856 had the following issue. That is, it was found that the junction body in PCT Japanese Translation Patent Publication No. 2012-520856 had high water solubility because a hydrophilic sulfonate group was bonded to a highly hydrophobic aromatic ring in the structure of the cyanine dye portion and was promptly discharged from the blood so as to indicate low tumor accumulation.

### SUMMARY OF THE INVENTION

The present invention in its first aspect provides a compound or a salt thereof as specified in claims 1 to 3 and 5 to 8.

The present invention in its second aspect provides a compound or a salt thereof as specified in claims 4 to 8.

The present invention in its third aspect provides a photoacoustic imaging contrast agent as specified in claims 9.

Further features of the present invention will become apparent from the following description of exemplary embodiments.

### DESCRIPTION OF THE EMBODIMENTS

The embodiments according to the present invention will be described below, although the present invention is not limited to these.

### Cyanine dye-polyethylene glycol covalent compound

A compound or a salt thereof according to the present embodiment is represented by any one of Formulae (1), (5), (6), and (7) below. In the present specification, the salt can be a pharmaceutically acceptable salt. 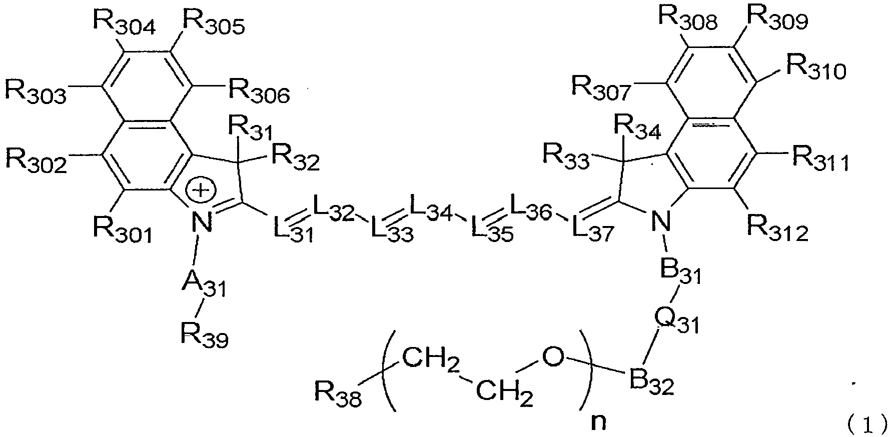 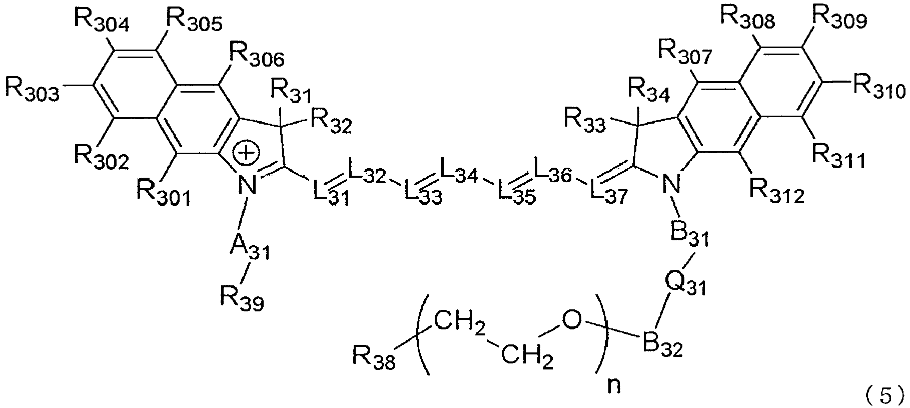 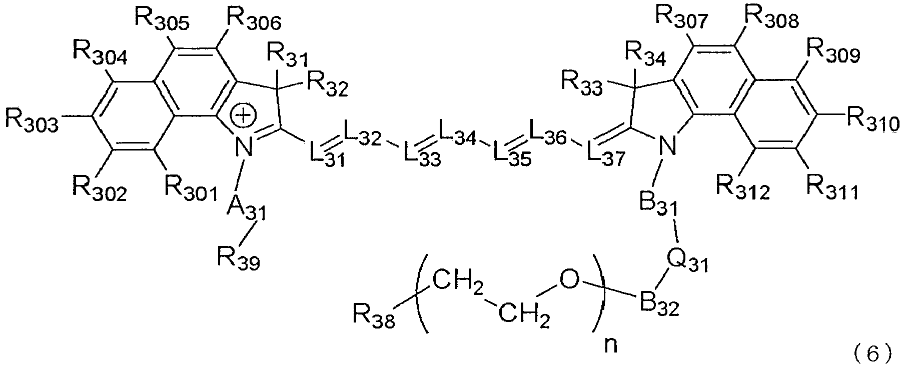 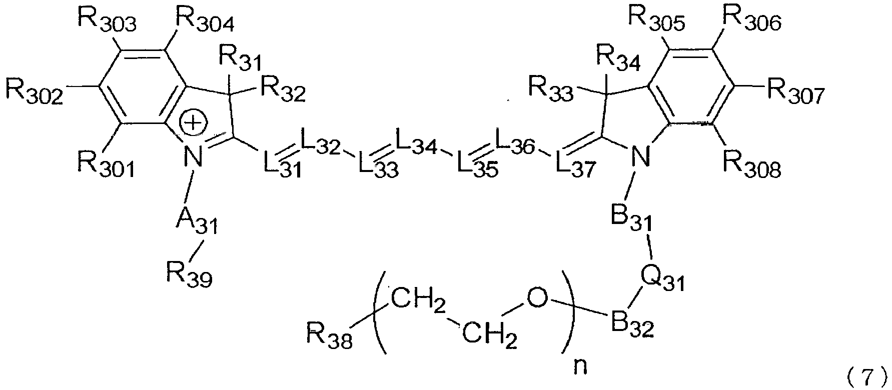 (in Formulae (1), (5), (6), and (7) above, each of R₃₀₁ to R₃₁₂ represents a hydrogen atom, a halogen atom, PO₃T₃₀₁, a substituted or unsubstituted phenyl group, thiophene group, pyridinyl group, or a straight chain or branched alkyl group having a carbon number of 1 to 18, T₃₀₁ represents any one of a hydrogen atom, a sodium atom, and a potassium atom, each of R₃₁ to R₃₄ represents a hydrogen atom or a straight chain or branched alkyl group having a carbon number of 1 to 18,
each of A₃₁, B₃₁, and B₃₂ represents a straight chain or branched alkylene group having a carbon number of 1 to 18, each of L₃₁ to L₃₇ represents CH or CR₃₅, L₃₁ to L₃₇ may constitute a 4-membered ring or 6-membered ring, R₃₅ represents a straight chain or branched alkyl group having a carbon number of 1 to 18, a halogen atom, a substituted or unsubstituted phenyl group, pyridinyl group, benzyl group, ST₃₀₂, or a straight chain or branched alkylene group having a carbon number of 1 to 18, T₃₀₂ represents a straight chain or branched alkyl group having a carbon number of 1 to 18, a substituted or unsubstituted phenyl group, or a straight chain or branched alkylene group having a carbon number of 1 to 18, Q₃₁ represents any one of -CONT₃₁-, -NT₃₁CO-, -NT₃₁(C=O)NT₃₁-, -NT₃₁(C=S)NT₃₁-, -NT₃₁(C=O)O-, -O-, -S-, -S(=O)₂NT₃₁ -, -OP(=O) (OT₃₁)O-, -S-S-, -CT₃₁=N-, -CT₃₁=N-NH-, -CT₃₁=N-O-, -CT₃₁=N-NH-O-, -CONT₃₁-R₃₇-(C=O)O-, -CONT₃₁-R₃₇-CONT₃₁-, and a group represented by Formula (2) or Formula (3) below,
T₃₁ represents a hydrogen atom or an alkyl group having a carbon number of 1 to 5, R₃₇ represents any one of -CH(CO₂T₃₇)-, -CH(CH₂CO₂T₃₇)-, and a straight chain or branched alkylene group having a carbon number of 1 to 18, T₃₇ represents any one of a hydrogen atom, a sodium atom, a potassium atom, and an alkyl group having a carbon number of 1 to 5, R₃₈ represents any one of -H, -OCH₃, -NH₂, -OH, -CO₂T₃₈, -S(=O)₂OT₃₈. -P(=O)(OT₃₈)₂, and -OP(=O) (OT₃₈)₂, T₃₈ represents any one of a hydrogen atom, a sodium atom, and a potassium atom,
R₃₉ represents any one of -H, -OCH₃, -NH₂, -OH, -CO₂T₃₉, -P(=O) (OT₃₉)₂, -CONH-CH (CO₂T₃₉)-CH₂(C=O)OT₃₉, -CONH-CH (CO₂T₃₉)-CH₂CH₂(C=O)OT₃₉, and -OP(=O)(OT₃₉)₂, T₃₉ represents any one of a hydrogen atom, a sodium atom, and a potassium atom, and
n represents an integer of 1 to 2,500)

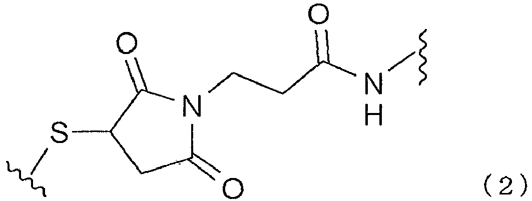

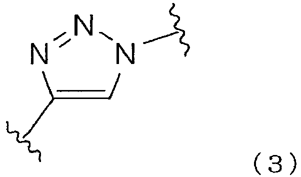

As described above, the compound represented by any one of Formulae (1), (5), (6), and (7) does not have a highly hydrophilic sulfonate group in the aromatic ring and, therefore, it is possible that the entirety of the compound has hydrophobicity and is not discharged from the blood promptly. Consequently, high tumor accumulation is exhibited.

In the present embodiment, Q₃₁ can be -CONH-, R₃₈ can be -OCH₃, R₃₉ can be -COOH, and each of R₃₀₁ to R₃₁₂ can be a hydrogen atom.

The molecular weight of the compound or a salt thereof according to the present embodiment is preferably 5,000 or more and 100,000 or less, more preferably 10,000 or more and 45,000 or less, and particularly preferably 20,000 or more and 45,000 or less.

The compound or a salt thereof according to the present embodiment can be represented by Formula (4) below. 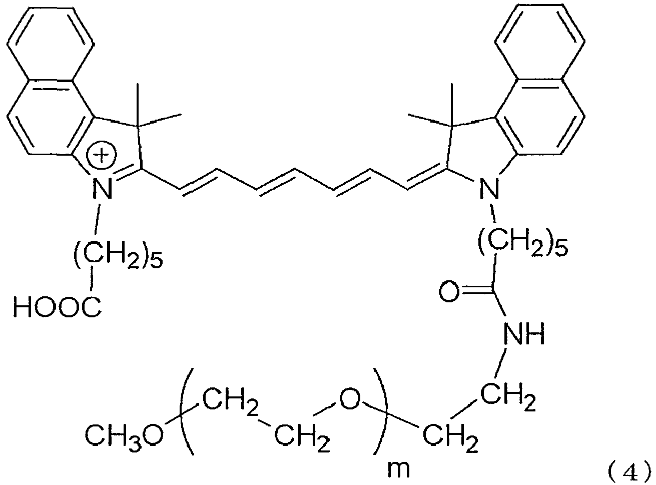 (m represents an integer of 1 to 2,500) Cyanine dye

The cyanine dye according to the present embodiment will be described.

The cyanine dye according to the present embodiment has a structure composed of a methine chain and nitrogen-containing five-membered rings bonded to both ends of the methine chain as a basic skeleton.

The structure of the cyanine dye can be represented by any one of Formulae (11), (15), (16), and (17) below.

The cyanine dye according to the present embodiment can be a compound that absorbs light with a wavelength within the range of 600 nm to 1,300 nm and that emits light. The cyanine dye according to the present embodiment has a molar adsorption coefficient at absorption maximum wavelength of preferably 10⁶ M⁻¹cm⁻¹ or more. 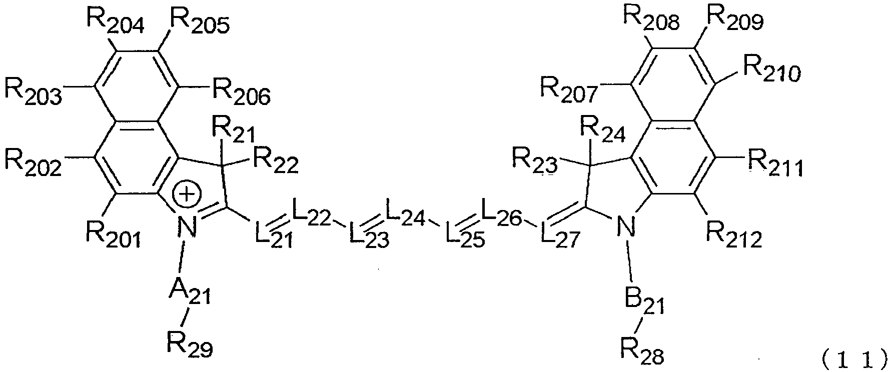 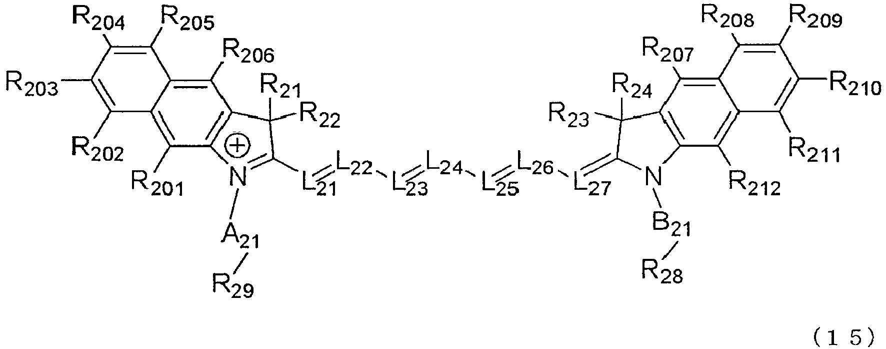 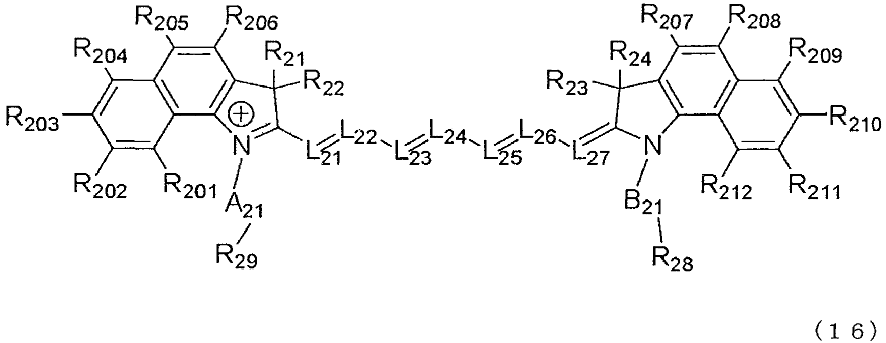 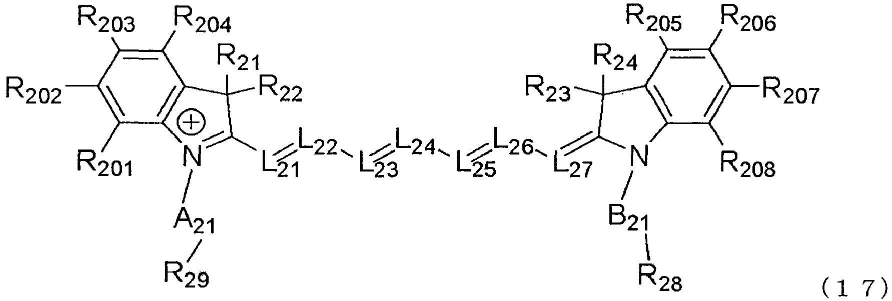 (in Formulae (11), (15), (16), and (17) above, each of R₂₀₁ to R₂₁₂ represents a hydrogen atom, a halogen atom, PO₃T₂₀₁, a benzene ring, a thiophene ring, a pyridine ring, or a straight chain or branched alkyl group having a carbon number of 1 to 18, T₂₀₁ represents any one of a hydrogen atom, a sodium atom, and a potassium atom, each of R₂₁ to R₂₄ represents a hydrogen atom or a straight chain or branched alkyl group having a carbon number of 1 to 18, each of A₂₁ and B₂₁ represents a straight chain or branched alkylene group having a carbon number of 1 to 18, each of L₂₁ to L₂₇ represents CH or CR₂₅, L₂₁ to L₂₇ may constitute a 4-membered ring or 6-membered ring, R₂₅ represents a straight chain or branched alkyl group having a carbon number of 1 to 18, a halogen atom, a benzene ring, a pyridine ring, a benzyl group, ST₂₀₂, or a straight chain or branched alkylene group having a carbon number of 1 to 18, T₂₀₂ represents a straight chain or branched alkyl group having a carbon number of 1 to 18, a benzene ring, or a straight chain or branched alkylene group having a carbon number of 1 to 18, R₂₈ represents any one of -H, -OCH₃, -NH₂, -OH, -CO₂T₂₈, -P(=O)(OT₂₈)₂, -CONH-CH(CO₂T₂₈)-CH₂(C=O)OT₂₈, -CONH-CH(CO₂T₂₈)-CH₂CH₂(C=O)OT₂₈, and -OP(=O)(OT₂₈)₂, T₂₈ represents any one of a hydrogen atom, a sodium atom, and a potassium atom,
R₂₉ represents any one of -H, -OCH₃, -NH₂, -OH, -CO₂T₂₉,
-S(=O)₂OT₂₉, -P(=O)(OT₂₉)₂, -CONH-CH (CO₂T₂₉)-CH₂(C=O)OT₂₉,
-CONH-CH(CO₂T₂₉)-CH₂CH₂(C=O)OT₂₉, and -OP(=O)(OT₂₉)₂, T₂₉ represents any one of a hydrogen atom, a sodium atom, and a potassium atom, and
t represents an integer of 1 to 2,500)

R₂₈ can be -COOH and R₂₉ can be -COOH.

The cyanine dye according to the present embodiment can be represented by Formula (12) below.

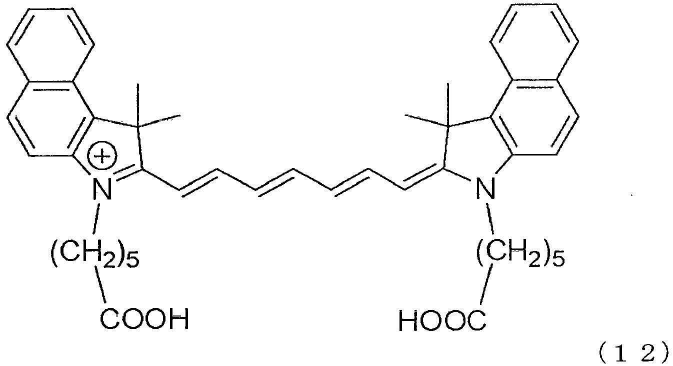

### Polyethylene glycol (PEG)

The molecular weight of the compound or a salt thereof according to the present embodiment is preferably 10,000 or more and 400,000 or less because dispersion occurs without aggregation.

The compound including polyethylene glycol according to the present embodiment may have not only a straight chain but also a branched structure. If the branched structure is included, dispersibility is enhanced.

A plurality of amino groups which can bond to the above-described cyanine dye may be included because it is possible to increase the number of bonds to cyanine dye per unit polyethylene glycol by bonding to a plurality of cyanine dyes.

Examples of the compound including polyethylene glycol according to the present embodiment include compounds represented by Formula (21) below. 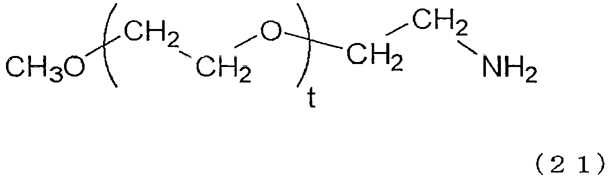 (in Formula (21) above, t is an integer of 1 to 2,500, and the molecular weight is preferably 5,000 or more and 40,000 or less, more preferably 10,000 or more, and particularly preferably 20,000 or more)

Other examples include SUNBRIGHT (registered trademark) PA Series (produced by NOF CORPORATION):
SUNBRIGHT MAPA-20H, SUNBRIGHT MEPA-12T, SUNBRIGHT MEPA-20T,
SUNBRIGHT MEPA-30T, and SUNBRIGHT MEPA-40T; SUNBRIGHT (registered trademark) EA Series (produced by NOF CORPORATION): SUNBRIGHT ME-100EA, SUNBRIGHT ME-200EA,
SUNBRIGHT ME-300EA, and SUNBRIGHT ME-400EA; SUNBRIGHT GL2-200PA (produced by NOF CORPORATION), SUNBRIGHT GL2-400PA (produced by NOF CORPORATION), SUNBRIGHT GL3-400PA 100U (produced by NOF CORPORATION), SUNBRIGHT PTE-400EA (produced by NOF CORPORATION), SUNBRIGHT PTE-400PA (produced by NOF CORPORATION), SUNBRIGHT HGEO-150PA (produced by NOF CORPORATION), SUNBRIGHT HGEO-400PA (produced by NOF CORPORATION), SUNBRIGHT PTE2-200MA2 (produced by NOF CORPORATION), SUNBRIGHT PTE-400MA2 (produced by NOF CORPORATION), and SUNBRIGHT PTE-200PA (produced by NOF CORPORATION). 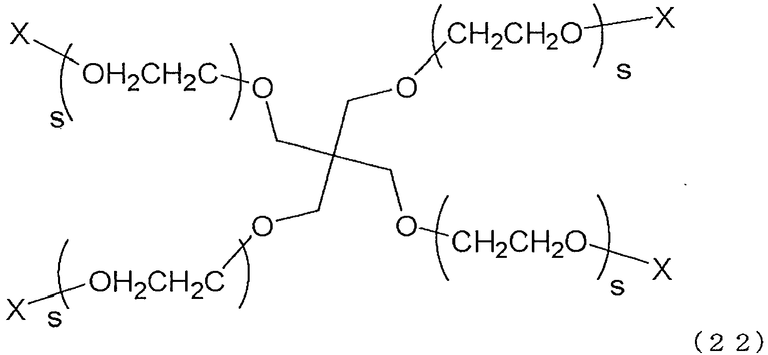
(in Formula (22) above, each of four degrees of polymerization s of the repetition unit is an integer of 1 to 2,500)

X can be -NH₂.

In Formula (22) above, the molecular weight is preferably 1,000 to 40,000.
Hereafter polyethylene glycol may be referred to as PEG. Photoacoustic imaging contrast agent

The photoacoustic imaging contrast agent according to the present embodiment includes the compound described above and a dispersion medium described later. Dispersion medium

Examples of the dispersion medium in the present embodiment include physiological saline, distilled water for injection, phosphate buffered saline, and glucose aqueous solution.

### Manufacturing method

A reaction process in the present embodiment includes a step for preparing the cyanine dye represented by any one of Formulae (11), (15), (16), and (17) above and the PEG derivative represented by Formula (21) above and a step for reacting the cyanine dye with the PEG derivative. Regarding the reaction process, examples of methods usable for the reaction between a carboxyl group and an amino group include a method in which a condensation agent is used, a method in which a salt is formed and condensation is performed by a dehydration reaction, a method in which a dehydration agent is used, and a method in which the carboxyl group is converted to an acid chloride so as to react with the amino group.

Regarding the condensation agent, carbodiimide condensation agents, phosphate condensation agents, and the like are used.

Examples of the carbodiimide condensation agents include N,N'-dicyclohexylcarbodiimide (DCC) and water-soluble carbodiimide (WSC).

The usage of the condensation agent is within the range of 0.1 or more times and preferably more than or equal to the usage of the compound represented by Formula (11) above on a mole basis. It is also possible to use the condensation agent by itself as a reaction solvent.

The usage of the compound represented by Formula (11) above in the reaction process in the present embodiment is within the range of preferably 0.1 to 50.0 times, further preferably 1.0 to 20.0 times, and particularly preferably 1.0 to 10.0 times the number of amino groups contained in the compound represented by Formula (21) on a mole basis. The reason is that as the amount of the compound represented by Formula (11) used in the reaction process decreases, a load of removing unreacted compound represented by Formula (11) in a sample decreases in a refining process.

### Organic solvent

There is no particular limitation regarding the material that may be used as the organic solvent used in the reaction process in the present embodiment as long as the compound represented by Formula (11) above is bonded to the compound represented by Formula (21) above.

Examples of the organic solvent used in the reaction process include hydrocarbons, e.g., hexane, cyclohexane, and heptane, ketones, e.g., acetone and methyl ethyl ketone, ethers, e.g., diethyl ether and tetrahydrofuran, halogenated hydrocarbons, e.g., dichloromethane, chloroform, carbon tetrachloride, dichloroethane, and trichloroethane, aromatic hydrocarbons, e.g., benzene and toluene, aprotic polar solvents, e.g., N,N-dimethyl formamide (hereafter may be abbreviated as DMF) and dimethylsulfoxide (hereafter may be abbreviated as DMSO), and pyridine derivatives. At least two types of these organic solvents may be used in combination.

Aprotic polar solvents, e.g., DMF and DMSO, and halogenated hydrocarbons, e.g., dichloromethane and chloroform, can be used because the compound represented by Formula (11) above is highly soluble in these organic solvents and the reaction is performed in a state in which the compound is sufficiently dispersed. The usage of the organic solvent in the reaction process is determined appropriately in accordance with the reaction conditions and the like.

Regarding the reaction process in the present embodiment, the reaction temperature is not specifically limited and is usually within the range of 0°C or higher and lower than or equal to the boiling point of the solvent. However, it is desirable that the reaction be performed at the most suitable temperature for the condensation agent employed.

Regarding the reaction process in the present embodiment, the reaction time is within the range of, for example, 1 to 48 hours.

### Refining process

Regarding the refining process in the present embodiment, the technique is not specifically limited as long as the junction body of the cyanine dye represented by Formula (11) above and the compound including polyethylene glycol represented by Formula (21) above is refined.

Examples of methods for isolating and refining the junction body obtained in the reaction process include normal phase chromatography using an organic solvent, gel filtration chromatography, ultrafiltration, and dialysis.

Examples of the organic solvent used in the refining process in the present embodiment include hydrocarbons, e.g., hexane, cyclohexane, and heptane, ketones, e.g., acetone and methyl ethyl ketone, ethers, e.g., diethyl ether and tetrahydrofuran, halogenated hydrocarbons, e.g., dichloromethane, chloroform, carbon tetrachloride, dichloroethane, and trichloroethane, aromatic hydrocarbons, e.g., benzene and toluene, aprotic polar solvents, e.g., N,N-dimethyl formamide and dimethylsulfoxide, pyridine derivatives, and alcohols, e.g., methanol and ethanol. At least two types of these solvents may be used in combination.

Halogenated hydrocarbons, e.g., dichloromethane and chloroform, and alcohols, e.g., methanol and ethanol, can be used.

### Examples

The present invention will be described below with reference to the examples. The present invention is not limited to these examples and the material, the compositional condition, the reaction condition, and the like are changed freely as long as a contrast agent having the same functions and advantages is obtained.

### Analyzing method

The structure of each compound obtained in each example described below was determined by using a ¹H-NMR (FT-NMR, Bruker DPX600, produced by Bruker, resonant frequency: 600 MHz) measurement apparatus.

### Photoacoustic characteristic evaluation method

Regarding the measurement of a photoacoustic signal, a sample was irradiated with pulse laser light, a photoacoustic signal from the sample was detected by using a piezoelectric element and was measured by a digital oscilloscope after being amplified by a high-speed preamplifier. Specific conditions were as described below. A titanium sapphire laser (produced by Lotis) was used as a light source. Regarding the conditions, the wavelength was 790 nm, the energy density was 12 mJ/cm², the pulse width was 20 nanoseconds, and the pulse repetition was 10 Hz. Model V303 (produced by Panametrics-NDT) was used as an ultrasonic transducer. Regarding the conditions, the center band was 1 MHz, the element size was φ 0.5, the measurement distance was 25 mm (non-focus), and the amplification was +30 dB (ultrasonic preamplifier Model 5682 produced by Olympus Corporation). A measurement container was a polystyrene cuvette, the optical path length was 0.1 cm, and the sampling capacitor was about 200 µL. The measurement was performed by using DPO4104 (produced by Tektronix Corporation), where the conditions were as described below.
trigger: photoacoustic light was detected by a photodiode
data acquisition: average of 128 times (128 pulses)

### Example A1

The cyanine dye represented by Formula (12) above was synthesized in accordance with Bioconjugate Chem., 2005, 16, P. 51-61.

Each of 28.2 mg of polyethylene glycol (SUNBRIGHT ME-050EA: produced by NOF CORPORATION, molecular weight: 5,000) having an amino group and 113.2 mg of cyanine dye represented by Formula (12) above was weighed and was dissolved in 4.0 mL of chloroform.

After 13.8 mg of 4-dimethylaminopyridine (hereafter may be abbreviated as DMAP) (produced by TOKYO KASEI KOGYO CO., LTD.) was added to the resulting solution, 23.5 mg of water-soluble carbodiimide, 1-ethyl-3-(3-dimethylaminopropyl)carbodiimide hydrochloride (hereafter may be abbreviated as WSC) (produced by Sigma-Aldrich Corporation) was added to the solution obtained by adding DMAP.

The resulting solution was agitated at room temperature for 24 hours.

Isolation and refining of 4 mL of reaction solution obtained by agitation was performed through dialysis by using a dialysis membrane (produced by Spectrum Laboratories, Inc., molecular cutoff of 15,000) and methanol as a solvent.

The solution recovered by isolation and refining was subjected to distillation by using an evaporator so as to remove the solvent and, thereafter, drying was performed by using a vacuum drier.

The compound obtained by drying was identified on the basis of ¹H-NMR measurement.

It was ascertained from the result of ¹H-NMR measurement that unreacted cyanine dye was not included in the finally obtained sample and, therefore, a target compound, that is, a cyanine dye-polyethylene glycol covalent compound, was obtained.

The resulting compound was dissolved in distilled water so as to obtain Photoacoustic imaging contrast agent (A-1).

### Example A2

Photoacoustic imaging contrast agent (A-2) was obtained by the reaction at the same molar ratio as in Example A1 and by the refining, in which the polyethylene glycol having an amino group was substituted with SUNBRIGHT ME-100EA (produced by NOF CORPORATION, molecular weight: 10,000).

### Example A3

Photoacoustic imaging contrast agent (A-3) was obtained by the reaction at the same molar ratio as in Example A1 and the refining, where the polyethylene glycol having an amino group was substituted with SUNBRIGHT ME-200EA (produced by NOF CORPORATION, molecular weight: 20, 000).

### Example A4

Photoacoustic imaging contrast agent (A-4) was obtained by the reaction at the same molar ratio as in Example A1 and the refining, where the polyethylene glycol having an amino group was substituted with SUNBRIGHT ME-400EA (produced by NOF CORPORATION, molecular weight: 40, 000).

### Comparative example C1

Photoacoustic imaging contrast agent (C-1) was produced by dissolving the official non-pharmacopoeial reference standard Indocyanine green (produced by Pharmaceutical and Medical Device Regulatory Science Society of Japan) in distilled water.

### Quantitative measurement of tumor accumulation and blood concentration

In order to examine the tumor accumulation of each of the photoacoustic imaging contrast agents prepared in the above-described examples and the comparative example, the contrast agent was administered, via the caudal vein, to a tumor-bearing mouse into which cells of the colon 26 cell line had been transplanted. The administered amount was 13 nmol in terms of the amount of dye. Blood was taken from the cauda of the mouse 24 hours after the administration, and the mouse was euthanized with carbon dioxide gas. Thereafter, colon 26 tumor tissue was enucleated. The tumor tissue was transferred to a plastic tube, 1% TritonX-100 aqueous solution 1.25 times the tumor tissue in weight was added, and a homogenate was produced using a plastic pestle. Subsequently, the homogenate solution was subjected to a centrifugal operation (16,000 x G, 5 minutes, and 4°C) so as to recover supernatant fluid. Dimethyl sulfoxide (DMSO) (18 µL) was added to the supernatant fluid (2 µL). Regarding the blood, 1% TritonX-100 aqueous solution (11 µL) and DMSO (9 µL) were added to the blood (4 µL) taken. The fluorescent intensity of the homogenate solution and the blood on 48-well plates was measured by using ODYSSEY (registered trademark) CLx Infrared Imaging System (produced by LI-COR) and, thereby, the amounts of the compound in the tumor tissue and the blood of the present examples and comparative example was quantitated. The proportion (% injected dose: abbreviated as %ID) of the amount of migration of the dye into the tumor tissue relative to the administered amount (13 nmol of dye) per unit weight of the tumor tissue is denoted as the amount of tumor accumulation of the compound (%ID/g).

In the present specification, the thus determined amount of accumulation of the compound in the tumor tissue is denoted as T (%ID/g), the retentivity of the dye of the compound in blood is denoted as B (%ID/g), and the value produced by dividing the value of T by the value of B is denoted as an index T/B (tumor accumulation/retentivity in blood).

The calculation results of the photoacoustic signal intensity ratio, the tumor accumulation, the retentivity in blood, and T/B (tumor accumulation/retentivity in blood) of each contrast agent are shown in Table. In the table, the term "N.D" means that the measured item was below the detection threshold.

**Table**

| | Contrast agent (A-1) | Contrast agent (A-2) | Contrast agent (A-3) | Contrast agent (A-4) | Contrast agent (C-1) |
|---|---|---|---|---|---|
| Polyethylene glycol molecular weight | 5000 | 10000 | 20000 | 40000 | - |
| Photoacoustic signal intensity ratio | 1.5 | 2.1 | 2.7 | 4.7 | 1.0 |
| Tumor accumulation (%ID/g) | 2.0 | 7.4 | 21.5 | 11.5 | N.D |
| Retentivity in blood (%ID/g) | 0.5 | 2.1 | 12.2 | 22.2 | N.D |
| T/B (tumor accumulation/retentivity in blood) | 3.9 | 3.5 | 1.8 | 0.5 | - |

As is clear from the above-described results, the cyanine dye-polyethylene glycol covalent compound according to the present example was suitable for a photoacoustic imaging contrast agent.

According to the present invention, the junction body of the cyanine dye and the polyethylene glycol is made to have a structure in which the aromatic ring of the cyanine dye does not have a sulfonate group so as to maintain hydrophobicity and, thereby, is not discharged from the blood promptly. As a result, the tumor accumulation is enhanced.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A compound or a salt thereof represented by any one of Formulae (1), (5), (6), and (7): 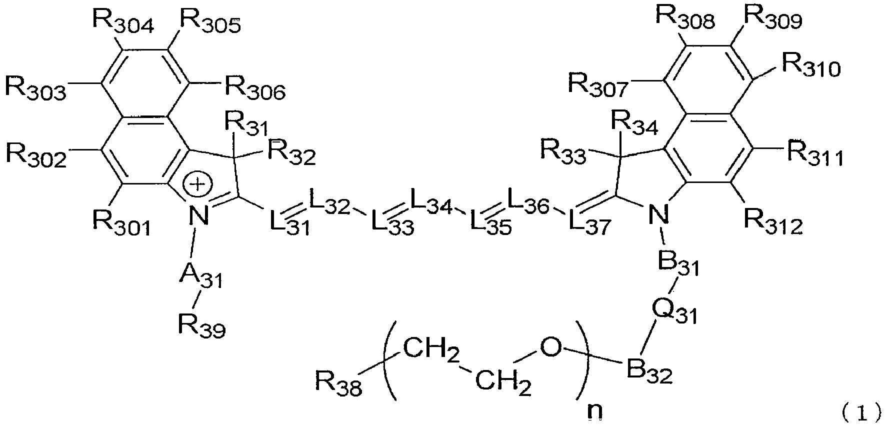 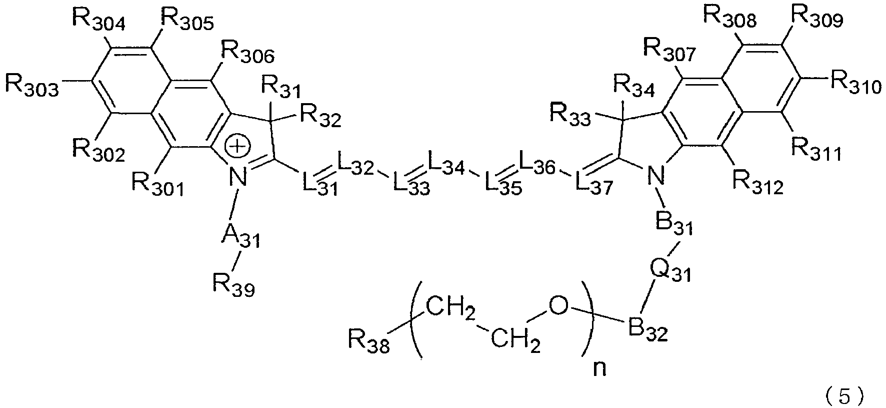 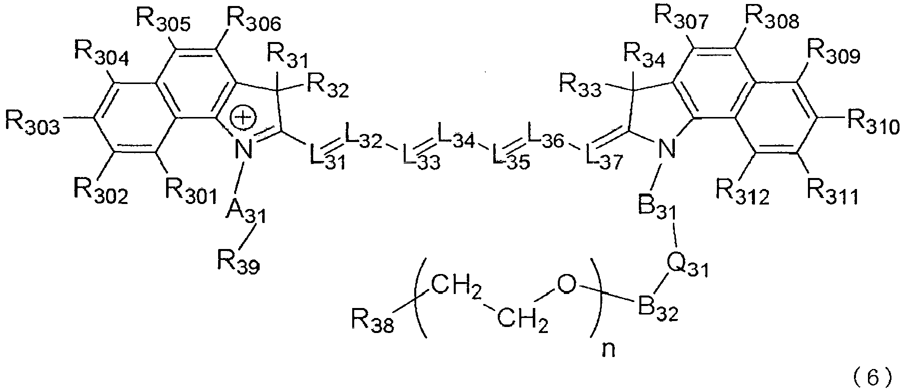 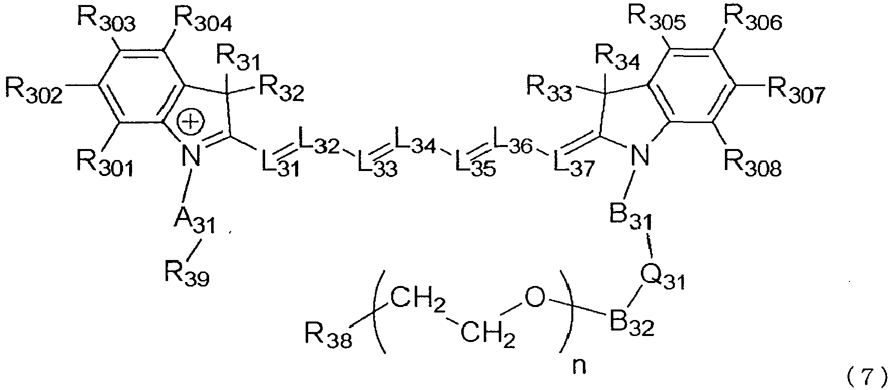 in Formulae (1), (5), (6), and (7), each of R₃₀₁ to R₃₁₂ represents a hydrogen atom, a halogen atom, PO₃T₃₀₁, a substituted or unsubstituted phenyl group, thiophene group, pyridinyl group, or a straight chain or branched alkyl group having a carbon number of 1 to 18, T₃₀₁ represents any one of a hydrogen atom, a sodium atom, and a potassium atom, each of R₃₁ to R₃₄ represents a hydrogen atom or a straight chain or branched alkyl group having a carbon number of 1 to 18,
each of A₃₁, B₃₁, and B₃₂ represents a straight chain or branched alkylene group having a carbon number of 1 to 18,
each of L₃₁ to L₃₇ represents CH or CR₃₅, R₃₅ represents a straight chain or branched alkyl group having a carbon number of 1 to 18, a halogen atom, a substituted or unsubstituted phenyl group, pyridinyl group, benzyl group, ST₃₀₂, or a straight chain or branched alkylene group having a carbon number of 1 to 18, T₃₀₂ represents a straight chain or branched alkyl group having a carbon number of 1 to 18, a substituted or unsubstituted phenyl group, or a straight chain or branched alkylene group having a carbon number of 1 to 18, Q₃₁ represents any one of -CONT₃₁-, -NT₃₁CO-,
-NT₃₁(C=O)NT₃₁-, -NT₃₁(C=S)NT₃₁-, -NT₃₁(C=O)O-, -O-, -S-,
-S(=O)₂NT₃₁-, -OP(=O) (OT₃₁)O-, -S-S-, -CT₃₁=N-, -CT₃₁=N-NH-,
-CT₃₁=N-O-, -CT₃₁=N-NH-O-, -CONT₃₁-R₃₇-(C=O)O-,
-CONT₃₁-R₃₇-CONT₃₁-, and a group represented by Formula (2) or Formula (3), 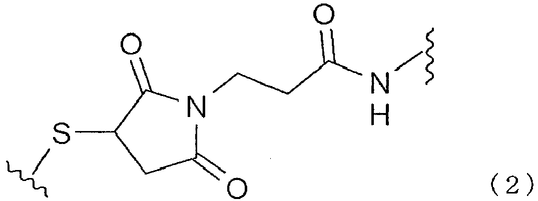 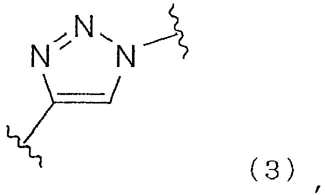 T₃₁ represents a hydrogen atom or an alkyl group having a carbon number of 1 to 5, R₃₇ represents any one of -CH(CO₂T₃₇)-, -CH(CH₂CO₂T₃₇)-, and a straight chain or branched alkylene group having a carbon number of 1 to 18, T₃₇ represents any one of a hydrogen atom, a sodium atom, a potassium atom, and an alkyl group having a carbon number of 1 to 5, R₃₈ represents any one of -H, -OCH₃, -NH₂, -OH, -CO₂T₃₈, -S(=O)₂OT₃₈, -P(=O)(OT₃₈)₂, and -OP(=O)(OT₃₈)₂, T₃₈ represents any one of a hydrogen atom, a sodium atom, and a potassium atom,
R₃₉ represents any one of -H, -OCH₃, -NH₂, -OH, -CO₂T₃₉, -P(=O)(OT₃₉)₂, -CONH-CH(CO₂T₃₉)-CH₂(C=O)OT₃₉, -CONH-CH(CO₂T₃₉)-CH₂CH₂(C=O)OT₃₉, and -OP(=O) (OT₃₉)₂, T₃₉ represents any one of a hydrogen atom, a sodium atom, and a potassium atom, and
n represents an integer of 1 to 2,500.

2. The compound or a salt thereof according to Claim 1, wherein Q₃₁ is -CONH-, R₃₈ is -OCH₃, and R₃₉ is -COOH.

3. The compound or a salt thereof according to Claim 1 or Claim 2, wherein each of R₃₀₁ to R₃₁₂ is a hydrogen atom

4. A compound or a salt thereof represented by Formula (4): 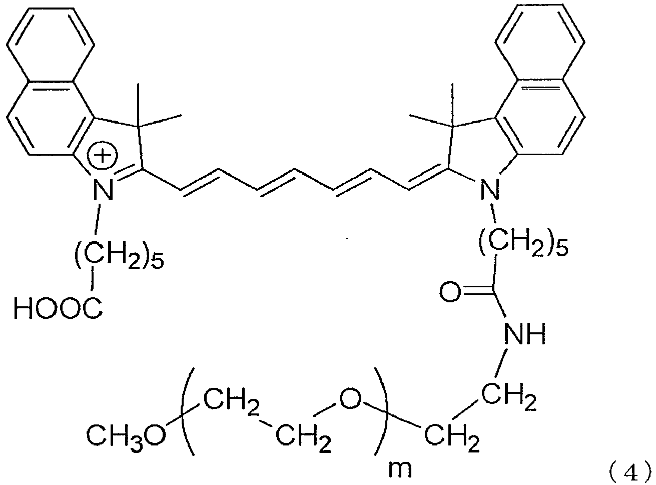 in Formula (4), m represents an integer of 1 to 2,500.

5. The compound or a salt thereof according to any one of Claims 1 to 4, comprising a molecular weight of 5,000 or more and 100,000 or less.

6. The compound or a salt thereof according to any one of Claims 1 to 5, comprising a molecular weight of 10,000 or more and 45,000 or less.

7. The compound or a salt thereof according to any one of Claims 1 to 6, comprising a molecular weight of 20,000 or more and 45,000 or less.

8. The compound or a salt thereof according to any one of Claims 1 to 7, wherein L₃₁ to L₃₇ constitute a 4-membered ring or 6-membered ring.

9. A photoacoustic imaging contrast agent comprising the compound or a salt thereof according to any one of Claims 1 to 8 and a dispersion medium.
